# EUROPÄISCHE PATENTSCHRIFT

(11) **EP 1 867 310 B1**
(45) Veröffentlichungstag und Bekanntmachung des Hinweises auf die Patenterteilung: **19.10.2011**
(21) Anmeldenummer: 07011716.3
(22) Anmeldetag: 14.06.2007
(51) Int. Cl.: A61F 13/04, A61F 13/10, A61F 13/15

(54) **Hygieneprodukt**
Hygiene product
Produit hygiénique

(30) Priorität: 16.06.2006 DE 202006009469 U
(43) Veröffentlichungstag der Anmeldung: 19.12.2007
(73) Patentinhaber: W. Pelz GmbH & Co. KG, 23812 Wahlstedt/Holstein (DE)
(72) Erfinder:
(74) Vertreter: Siemons, Norbert

(56) Entgegenhaltungen:
- EP-A- 0 622 064
- US-A- 4 718 898
- US-A- 4 731 066
- US-A- 4 778 458

## Beschreibung

Die Erfindung bezieht sich auf ein Verfahren zum Herstellen eines Hygieneproduktes für Ausscheidungen des menschlichen Körpers.

Hygieneprodukte für Ausscheidungen des menschlichen Körpers sind beispielsweise Binden, Dickbinden, Inkontinenzeinlagen, Inkontinenzvorlagen, Inkontinenzpants, Slipeinlagen, (Wöchnerinnen-) Vorlagen, Höschen-Windeln und Stilleinlagen. Bekannte Hygieneprodukte weisen eine Wäscheschutzschicht und eine darauf angeordnete Absorptionsschicht auf, die an der von der Wäscheschutzschicht abgewandten Seite mit einer flüssigkeitsdurchlässigen Abdeckung versehen sein kann. Das Hygieneprodukt wird mit der von der Wäscheschutzschicht abgewandten Seite an den Körper des Menschen angelegt, dort wo Körperflüssigkeit austritt. Die Körperflüssigkeit gelangt - gegebenenfalls durch die Abdeckung hindurch - in die Absorptionsschicht hinein und wird von der Wäscheschutzschicht von der Kleidung der Benutzerin abgehalten.

Bei herkömmlichen Hygieneprodukten wird die Wäscheschutzschicht von einer Folie aus Kunststoff gebildet. Die Folie an der körperabgewandten Außenseite des Hygieneproduktes wird bei der Benutzung vielfach als unangenehm empfunden.

Ferner sind Hygieneprodukte bekannt, bei denen die Wäscheschutzschicht durch ein von einer Folie kaschiertes Nonwoven gebildet ist. Die Folie ist verhältnismäßig dick, damit sie beim kontinuierlichen Herstellungsprozeß des Laminates, bei dem sie von einer Rolle abgewickelt wird, nicht reißt. In der Regel sind die verschiedenen Lagen des Laminates so miteinander verbunden, daß sie sich nicht lösen und gegeneinander verschieben können. Trotzdem kann eine verhältnismäßig dicke Folie, die auflaminiert ist, als störend empfunden werden. Falls sich die Lagen gegeneinander verschieben, kann das Hygieneprodukt beim Tragen rascheln, was ebenfalls als störend empfunden wird.

Ferner bekannt ist der Einsatz von hydrophoben Spinnvliesen als Wäscheschutzschicht. Diese Spinnvliese sind luftdurchlässig und sperren Flüssigkeit. Sie können jedoch unter erhöhtem Wasserdruck oder bei Einsatz von Duftölen in Verbindung mit Emulgatoren durchlässig werden.

US 4 731 066 offenbart ein Hygieneprodukt, z.B. eine Binde, mit einem flüssigkeitsundurchlässigen Film bestehend aus einem Schmelzklebstoff aufgetragen auf einem Nonwoven und einer Absorptionsschicht bestehen aus Zellulosefasern. Der flüssigkeitsundurchlässige Film besteht aus einer einzigen Schicht des Schmelzklebstoffes.

US-A-4 778 458 offenbart ein Hygieneprodukt, z.B. eine Windel, mit einem flüssigkeitsundurchlässigen Film bestehend aus einem Schmelzklebstoff, der auf der Absorptionsschicht aufgetragen ist. Auch bei diesem Hygieneprodukt besteht der flüssigkeitsundurchlässige Film aus einer einzigen Schicht des Schmelzklebstoffes.

US-A-4 718 898 offenbart ein Hygieneprodukt, z.B. eine Binde, mit einer flüssigkeitsundurchlässigen Schutzschicht bestehend aus einem Nonwoven auf dem ein Schmelzklebstoff aufgetragen ist und einer Absorptionsschicht. Die Beschichtung aus Hotmelt kann durch eine, zwei oder mehr Klebstoffaufträge erzeugt werden. Dieses Dokument beschreibt jedoch nicht, die unterste Schicht mit geringem Flächengewicht auf das Nonwoven aufzubringen, so dass sie nur geringfügig in den Nonwoven eindringt und es gerade zu einer guten Verankerung mit dem Nonwoven kommt. Auch ist darin nicht beschrieben, dass die folgenden Schichten mit der untersten Schicht verschmelzen und von dieser an einem Eindringen in das Nonwoven gehindert werden.

EP-A-0 622 064 offenbart ein Hygieneprodukt bestehend aus einer flüssigkeitsundurchlässigen Schaumschicht welche mittels einem Schmelzklebstoff mit einer Absorptionsschicht gebunden ist. Der Schmelzklebstoff besteht lediglich aus einer einzigen Schicht.

Davon ausgehend liegt der Erfindung die Aufgabe zugrunde, ein Verfahren zum Herstellen eines Hygieneproduktes zur Verfügung zu stellen, das zuverlässig zur Wäscheseite hin abgedichtet ist und günstigere Benutzungseigenschaften aufweist.

Die Aufgabe wird durch ein Verfahren mit den Merkmalen des Anspruches 1 gelöst. Vorteilhafte Ausgestaltungen des Verfahrens sind in den Unteransprüchen angegeben.

Das erfindungsgemäße Verfahren zum Herstellen eines Hygieneproduktes zum Aufnehmen von Ausscheidungen des menschlichen Körpers hat eine Wäscheschutzschicht umfassend ein Laminat aus einem Nonwoven und einem auf einer Innenseite des Nonwovens angeordneten geschlossenen, flüssigkeitsundurchlässigen Film aus einem Schmelzklebstoff und eine auf dem Film angeordnete Absorptionsschicht ist **dadurch gekennzeichnet, dass** der Film aus mehreren Schichten aus Schmelzklebstoff auf die Innenseite des Nonwovens aufgebracht wird, wobei die unterste Schicht, die direkten Kontakt mit dem Nonwoven hat, aufgrund ihres geringen Flächengewichtes nur geringfügig in das Nonwoven eindringt, so dass es gerade zu einer guten Verankerung mit dem Nonwoven kommt, und die folgenden Schichten mit der untersten Schicht verschmelzen.

Bei dem nach dem erfindungsgemäßenVerfahren hergestellten Hygieneprodukt weist die Wäscheschutzschicht ein Laminat auf, das eine Verbindung aus einem Nonwoven und einem geschlossenen, flüssigkeitsundurchlässigen Film aus einem Schmelzklebstoff ist. Der Schmelzklebstoff (auch "Hotmelt" genannt) ist bei der Herstellung aufgrund Erwärmung im schmelzflüssigen Zustand auf das Nonwoven aufgebracht, so daß er nach dem Abkühlen bzw. Abbinden auf dem Nonwoven einen geschlossenen, flüssigkeitsundurchlässigen Film bildet, der am Nonwoven anhaftet. Dieser flüssigkeitsundurchlässige Film kann äußerst dünn sein bzw. ein geringes Flächengewicht haben, wobei das Flächengewicht von den Flüssigkeitsbelastungen abhängen kann, denen das Hygieneprodukt standhalten muß. Das Laminat ist somit flüssigkeitsdicht. Es kann so ausgelegt sein, daß es Flüssigkeit auch dann nicht durchläßt, wenn diese unter erhöhtem Druck ansteht bzw. Duftöle in Verbindung mit Emulgatoren zum Einsatz kommen. Der Film vermittelt kein unangenehmes Tragegefühl, da er an der Innenseite des Nonwovens angeordnet ist. Die körperabgewandte Außenseite des Hygieneproduktes hat aufgrund des dort angeordneten Nonwovens eine textile Oberfläche und wird als angenehm empfunden. Da Film und Nonwoven zu einem einteiligen Laminat miteinander verbunden sind, werden Relativbewegungen und unangenehme Raschelgeräusche vermieden. Dem Tragekomfort kommt zudem zugute, daß das Laminat eine äußerst geringe Stärke und damit ein geringes Flächengewicht (auch "Grammatur" genannt) aufweisen kann. Dies ermöglicht die Herstellung von Hygieneprodukten mit geringer Stärke, die beim Tragen weniger stören. Da es möglich ist, den Schmelzklebstoff äußerst dünn bzw. mit geringem Flächengewicht aufzutragen, ohne daß das Laminat flüssigkeitsdurchlässig wird, ist das Laminat und damit das Hygieneprodukt sehr preisgünstig herstellbar. Besonders vorteilhaft und kostenmindernd für die Produktion ist, daß der Schmelzklebstoff "inline" bzw. bei der Produktion des Wäscheschutzes auf das Nonwoven aufgebracht werden kann. Ein weiterer Vorteil besteht darin, daß eine anschließend aufgebrachte Absorptionsschicht durch den abbindenden Schmelzklebstoff mit dem Laminat verbindbar ist. Dann ist der Schmelzklebstoff zugleich Wäscheschutz und Konstruktionskleber. Das Laminat ist zuverlässig dicht, hat eine stoffartige Oberfläche, kann verhältnismäßig dünn ausgeführt sein, ist geräuschmindernd und preisgünstig. Der Aufbau des Hygieneproduktes vermeidet Müll und schont Ressourcen. Hierdurch hat das Hygieneprodukt eine hohe Akzeptanz.

Ein geeigneter Schmelzklebstoff wird beispielsweise von der Firma H.B. Fuller Deutschland GmbH, An der Roten Bleiche 2-3, 21335 Lüneburg unter der Bezeichnung LUNATACK^{®} D 849AD56 ZP^{®} angeboten und vertrieben. Ein geeignetes Auftragssystem zum Auftragen des vorbezeichneten Schmelzklebstoffes wird beispielsweise von der Firma Nordson Engineering GmbH, Lilienthalstraße 6, 21337 Lüneburg angeboten und vertrieben.

Schmelzklebstoffe werden bislang bei (Damen-)Binden und Slipeinlagen für Klebestellen eingesetzt, die der Befestigung dieser Hygieneprodukte an der Wäsche der Benutzerin dienen. Die Klebstellen sind vor Benutzung durch eine Schutzschicht aus Silikonpapier abgedeckt, das vor Anbringung des Hygieneproduktes abgezogen wird. Die Klebestellen befinden sich jedoch an der körperabgewandten Außenseite des Hygieneproduktes und bedecken diese nur teilweise, so daß sie nicht geeignet wären, einen Flüssigkeitsdurchgang zu verhindern. Hierfür sind die besagten Produkte ohnehin mit einer der eingangs erwähnten Wäscheschutzschichten aus dem Stand der Technik versehen. Zudem ist der Schmelzklebstoff in dem Bereich der Klebestellen sehr dick aufgetragen, wobei das Flächengewicht über 15 g/m² beträgt, damit eine hinreichende Klebewirkung erzielt wird.

Gemäß einer Ausgestaltung hat der Film aus Schmelzklebstoff ein Flächengewicht von maximal 15 g/m². Ein Film mit einem Flächengewicht im genannten Bereich führt zu einem entsprechend dünnen Laminat und Hygieneprodukt und ist verhältnismäßig kostengünstig.

Der Film aus Schmelzklebstoff ist mindestens so dick, daß er eine geschlossene flüssigkeitsundurchlässige Schicht bildet. Das minimale Flächengewicht des Filmes kann insbesondere von den Eigenschaften des verwendeten Schmelzklebstoffes und des verwendeten Auftragssystemes, vom verwendeten Nonwoven und von seiner Bahngeschwindigkeit in der Produktion abhängen. Ferner von den Flüssigkeitsbelastungen, denen der Film im jeweiligen Hygieneprodukt standhalten muß, ohne Flüssigkeit durchzulassen. Für jede Festlegung dieser Parameter gibt es eine Mindeststärke bzw. ein minimales Flächengewicht des Filmes, dessen Unterschreitung dazu führt, daß der Schmelzklebstoff keinen geschlossenen, flüssigkeitsundurchlässigen Film auf dem Nonwoven bildet. Gemäß einer Ausgestaltung weist der Film mindestens dieses minimale Flächengewicht auf.

Gemäß einer Ausgestaltung beträgt das Flächengewicht des Filmes mindestens etwa 1 g/m².

Gemäß einer Ausgestaltung beträgt das Flächengewicht des Filmes etwa 2 bis 20 g/m².

Gemäß einer Ausgestaltung beträgt das Flächengewicht des Filmes etwa 3 bis 15 g/m².

Der Film umfasst mehrere aufeinander aufgebrachte bzw. aufeinander extrudierte Schichten aus Schmelzklebstoff. Der Auftrag in mehreren Schichten ermöglicht sehr geringe Auftragstärken bzw. Flächengewichte in jeder einzelnen Schicht, wobei die in den Schichten vorhandenen Fehlstellen von mindestens einer weiteren Schicht abgedeckt werden. In der Summe sind hierdurch sehr geringe Auftragsgewichte bzw. Flächengewichte realisierbar. Die Minimierung des Auftragsgewichtes des Schmelzklebstoffes ist sowohl hinsichtlich der Produkteigenschaften als auch der Kosten sehr vorteilhaft. Wenn nur eine Schicht aufgetragen wird, kann das Flächengewicht des aufgetragenen Schmelzklebstoffes eine Grenze nicht unterschreiten, unter der zunehmen Fehlstellen auftreten und eine Undurchlässigkeit bewirken, welche die Schutzwirkung des Hygieneproduktes herabsetzt. Durch eine zweite oder dritte Schicht des Schmelzklebstoffes, auch wenn sie jeweils sehr dünn ist und infolgedessen Fehlstellen aufweist, kann eine insgesamt geschlossene Barriereschicht erzielt werden. Die Wahrscheinlichkeit, daß sich Fehlstellen überlagern und zu einer Undichtigkeit des Filmes führen, ist äußerst gering. In der Regel werden sich die Fehlstellen versetzt in der Fläche befinden. Gemäß einer bevorzugten Ausgestaltung umfaßt der Film mehrere aufeinander aufgebrachte bzw. aufeinander extrudierte Schichten aus Schmelzklebstoff, wobei jede Schicht Fehlstellen aufweist. Auf diese Weise kann das Flächengewicht extrem reduziert werden, ohne daß ein durchlässiger Film entsteht. Gemäß einer Ausgestaltung beträgt das Flächengewicht jeder Schicht etwa 0,5 bis 10 g/m².

Der geschlossene, flüssigkeitsundurchlässige Film erstreckt sich unterhalb der Absorptionsschicht so, daß aus dieser keine Flüssigkeit durch das Nonwoven hindurchtreten kann. Hierfür überdeckt der Film im wesentlichen die gesamte Unterseite der Absorptionsschicht. Randbereiche des Nonwovens müssen nicht zwingend mit dem Film bedeckt sein, z.B. wenn sich die Absorptionsschicht nicht bis in die Randbereiche erstreckt und/oder eine flüssigkeitsdurchlässige Abdeckung auf der Außenseite der Absorptionsschicht mit den Randbereichen des Nonwovens verbunden ist. Gemäß einer Ausgestaltung bedeckt der Film das Nonwoven vollflächig. Hierbei können sich z.B. Absorptionsschicht und Nonwoven vollflächig überdecken. Falls die Oberfläche der Absorptionsschicht kleiner ist als das Nonwoven, sind bevorzugt nur die Bereiche des Nonwovens mit dem Film abgedeckt, die die Absorptionsschicht überdecken, nicht jedoch die freien Randbereiche. So erstreckt sich der Film beispielsweise nicht in den Randversiegelungsbereich oder in Bereiche von seitlichen Flügeln, die das Hygieneprodukt aufweisen kann, um das Hygieneprodukt im Steg eines Höschens zu befestigen.

An der Außenseite des Nonwovens ist kein durch das Nonwoven hindurchgedrungener Schmelzklebstoff vorhanden.

Erfingdungsgemäß wird ein Durchdringen des Schmelzklebstoffes von der Innenseite zur Außenseite des Nonwovens dadurch vermieden, dass ein Film aus mehreren aufeinander aufgebrachten bzw. aufeinander extrudierten Schichten aus Schmelzklebstoff aufgebracht wird. Die unterste Schicht, die direkten Kontakt mit dem Nonwoven hat, dringt aufgrund ihres geringen Flächengewichtes nur geringfügig in das Nonwoven ein, so daß es gerade zu einer guten Verankerung mit dem Nonwoven kommt. Die folgenden Schichten verschmelzen mit der untersten Schicht und werden von dieser an einem Eindringen in das Nonwoven gehindert.

Ein Nonwoven ist grundsätzlich ein textiles Flächengebilde aus verfestigten Fasern. Das Laminat kann insbesondere ein wasserstrahlverfestigtes oder ein nadelverfestigtes oder ein bindemittelverfestigtes Nonwoven aufweisen. Gemäß einer Ausgestaltung weist das Laminat ein thermoverfestigtes Nonwoven auf. Ein thermoverfestigtes Nonwoven umfaßt Kunststoffasern, die bei der Herstellung des Nonwovens zumindest an der Oberfläche aufgeschmolzen werden, so daß nach dem Abkühlen an den Kontaktstellen feste Verbindungen entstehen. Die Verfestigung wird durch ein punktförmiges Verschweißen der Fasern im wesentlichen über den gesamten Querschnitt des Nonwovens bewirkt. Die Struktur der thermoverfestigten Nonwoven ist durch die Verschweißungen der Fasern geprägt. Thermoverfestigte Nonwoven werden z.B. hergestellt, in dem als Ballenware bereitgestellte Polypropylen-Fasern aufgelöst, als Flor abgelegt und durch temperierte Kalander hindurchgeführt werden, wobei die Fasern an- bzw. verschmelzen und sich nach Abkühlen ein thermoverfestigtes Nonwoven aus Polypropylenfasern bildet.

Gemäß einer anderen Ausgestaltung ist das Nonwoven ein Spinnvlies. Ein Spinnvlies wird z.B. hergestellt, indem mittels einer Spinndüse aus aufgeschmolzenem Kunststoff dünne Fasern hergestellt werden, die von einem starken Luftstrom auf einem Band abgelegt werden, wo sie durch thermisches Verbinden miteinander verbunden werden.

Gemäß einer Ausgestaltung ist der Schmelzklebstoff ein druckempfindlicher Schmelzklebstoff. Derartige Schmelzklebstoffe werden gemäß Obigem für Klebestellen zur Befestigung an der Kleidung an den körperabgewandten Seiten von (Damen-)Binden und Slipeinlagen eingesetzt.

Gemäß einer Ausgestaltung ist der Film aus Schmelzklebstoff durch den Schmelzklebstoff mit der Absorptionsschicht verklebt. Der Schmelzklebstoff dient somit zugleich der Abdichtung und dem Zusammenhalt verschiedener Schichten des Hygieneproduktes.

Die Absorptionsschicht kann verschieden beschaffen sein. Gemäß einer Ausgestaltung umfaßt sie ein Fluffkissen oder ein Airlaid mit oder ohne enthaltenen Superabsorber. Bei dem Fluffkissen handelt es sich z.B. um zu einem Kissen geformten Zellulosefasern. Ein Airlaid besteht z.B. aus vom Luftstrom auf einem Sieb abgelegten und bei Vakuum von unten unter Verfestigung durch Bindemittel oder Aufheizen bei enthaltenen Schmelzfasern hergestelltem Zellulosematerial. Das Airlaid kann auch superabsorbierende Substanzen, wie Superabsorber (SAP) enthalten.

Gemäß einer Ausgestaltung umfaßt die Absorptionsschicht Zellulosefasern und/oder Baumwollfasern und/oder Viskosefasern.

Grundsätzlich ist es möglich, daß die Absorptionsschicht direkt mit der Oberseite an den Körper der Benutzerin angelegt wird. Ferner ist es möglich, daß die Fasern an der Außenseite der Absorptionsschicht bzw. die Absorptionschicht in Oberflächennähe verdichtet (z.B. durch Prägen) oder wasservemadelt oder anderweitig verfestigt sind, um einem Austritt von Fasern an der Außenseite entgegenzuwirken bzw. ein angenehmes Tragegefühl zu vermitteln. Gemäß einer Ausgestaltung ist auf der Außenseite der Absorptionsschicht eine flüssigkeitsdurchlässige Abdeckung angeordnet. Die Abdeckung ist z.B. aus einem Material als die Absorptionsschicht mit einem geeigneten Griff, das sich auf der Haut der Benutzerin gut anfühlt. Ferner ist es möglich, durch geeignete Ausführung der Abdeckung den Flüssigkeitsstrom in die Absorptionsschicht hineinzusteuern und zu verhindern, daß Flüssigkeit aus der Absorptionsschicht durch die Abdeckung hindurch nach außen tritt. Die flüssigkeitsdurchlässige Abdeckung ist z.B. durch einen stellenweise oder punktuell aufgebrachten Kleber mit der Außenseite und/oder dem Rand der Absorptionsschicht verbunden. Zusätzlich oder statt dessen kann die flüssigkeitsdurchlässige Abdeckung um die Ränder in der Absorptionsschicht geschlagen und zumindest randseitig unterhalb der Absorptionsschicht mit dem Film aus Schmelzklebstoff an der Oberseite der Wäscheschutzschicht verbunden sein. Hierbei kann der Film aus Schmelzklebstoff zugleich einen Konstruktionskleber bilden, der die flüssigkeitsdurchlässige Abdeckung fixiert.

Gemäß einer Ausgestaltung ist die Abdeckung eine Lochfolie und/oder ein perforiertes oder nicht perforiertes Nonwoven. Eine Lochfolie ist eine mit einer Vielzahl Löchern versehene Folie aus einem Kunststoff. Durch die Gestaltung der Löcher bzw. Perforation kann gegebenenfalls die Richtung des Flüssigkeitsstromes gesteuert werden.

Gemäß einer Ausgestaltung sind verschiedene Schichten des Hygieneproduktes durch Klebstoff (z.B. Schmelzklebstoff) miteinander verbunden. Gemäß einer weiteren Ausgestaltung sind verschiedene Schichten des Hygieneproduktes miteinander versiegelt und/oder verprägt. Diese Ausgestaltungen betreffen die Verbindung des Laminats mit der Absorptionsschicht und/oder des Laminats und/oder der Absorptionsschicht mit der Abdeckung. Es sind auch Kombinationen der verschiedenen Verbindungsweisen möglich. Auch ist es möglich, die Absorptionsschicht ohne besondere zusätzliche Verbindung zwischen der Wäscheschutzschicht und der randseitig mit dieser verbundenen Abdeckung zu fixieren.

Gemäß einer Ausgestaltung ist das Hygieneprodukt eine Binde, Dickbinde, Inkontinenzeinlage, Inkontinenzvorlage, Inkontinenzpant, Slipeinlage, (Wöchnerinnen-) Vorlage, (Höschen-)Windel oder Stilleinlage.

Die Erfindung wird nachfolgend anhand der anliegenden Zeichnung eines Ausführungsbeispieles erläutert, die eine Stilleinlage in einem Querschnitt zeigt.

Die Stilleinlage 1 hat im wesentlichen einen schalenförmigen Körper 2 mit einem nach außen leicht abgewinkelten, umlaufenden Rand 3. In der Mitte ist sie mit einer Auswölbung 4 für die Aufnahme eines Brustwarzennippels versehen.

Die Stilleinlage 1 weist außen ein Nonwoven 5 auf, das an der Innenseite einen geschlossenen Film 6 aus einem Schmelzklebstoff trägt. Das Nonwoven 5 und der Film 6 aus Schmelzklebstoff sind zu einem Laminat miteinander verbunden, das eine Wäscheschutzschicht 7 bildet.

Auf der Innenseite der Wäscheschutzschicht 7 ist eine dickere Absorptionsschicht 8 aus Zellulose angeordnet. Die Absorptionsschicht ist mit dem Film 6 der Wäscheschutzschicht 7 verklebt.

Auf der Außenseite der Absorptionsschicht 8 ist ein Nonwoven als Abdeckung 9 angeordnet, die z.B. punktuell mit der Absorptionsschicht 8 verklebt ist. Die Abdeckung 9 ist flüssigkeitsdurchlässig.

Bei Anwendung tritt Körperflüssigkeit durch die flüssigkeitsdurchlässige Abdeckung 9 in die Absorptionsschicht 8 ein. Die Wäscheschutzschicht 7 bzw. der Film 6 verhindert, daß Körperflüssigkeit aus der Absorptionsschicht 8 durch das Nonwoven 5 hindurch auf die Außenseite und damit in die Wäsche der Benutzerin gelangt.

Eine Binde oder Slipeinlage kann mit einem entsprechenden Schichtaufbau aus flachen Bahnabschnitten der genannten Materialien gebildet werden.

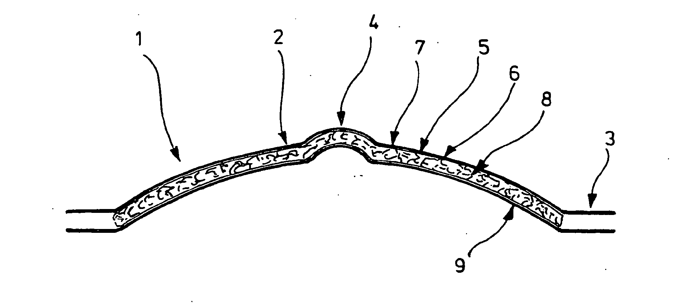

## Patentansprüche

1. Verfahren zum Herstellen eines Hygieneproduktes zum Aufnehmen von Ausscheidungen des menschlichen Körpers mit einer Wäscheschutzschicht (7) umfassend ein Laminat aus einem Nonwoven (5) und einem auf der Innenseite des Nonwovens angeordneten geschlossenen, flüssigkeitsundurchlässigen Film (6) aus einem Schmelzklebstoff und eine auf dem Film (6) angeordnete Absorptionsschicht (8), **dadurch gekennzeichnet, dass** ein Film (6) aus mehreren Schichten auf Schmelzklebstoff auf die Innenseite des Nonwovens (5) aufgebracht wird; wobei die unterste Schicht, die direkten Kontakt mit dem Nonwoven hat, aufgrund ihres geringen Flächengewichtes nur geringfügig in das Nonwoven (5) eindringt, so dass es gerade zu einer guten Verankerung mit dem Nonwoven (5) kommt, und die folgenden Schichten mit der untersten Schicht verschmelzen.

2. Verfahren gemäß Anspruch 1, bei dem der Film (6) aus Schmelzklebstoff ein Flächengewicht von maximal 15 g/m² aufweist.

3. Verfahren nach Anspruch 1 oder 2, bei dem das Flächengewicht des Filmes (6) mindestens 1 g/m² beträgt.

4. Verfahren nach einem der Ansprüche 1 bis 3, bei dem das Flächengewicht des Filmes etwa 3 bis 15 g/m² beträgt.

5. Verfahren nach einem der Ansprüche 1 bis 4, bei dem der Film (6) das Nonwoven (5) teilweise oder vollflächig bedeckt.

6. Verfahren nach einem der Ansprüche 1 bis 5, bei dem an der Außenseite des Nonwovens (5) kein durch das Nonwoven (5) hindurchgedrungener Schmelzklebstoff vorhanden ist.

7. Verfahren nach einem der Ansprüche 1 bis 6, bei dem das Nonwoven (5) ein thermoverfestigtes Nonwoven (5) ist.

8. Verfahren nach einem der Ansprüche 1 bis 7, bei dem das Nonwoven (5) ein Spinnvlies ist.

9. Verfahren nach einem der Ansprüche 1 bis 8, bei dem der Schmelzklebstoff des Films (6) ein druckempfindlcher Schmelzklebstoff ist.

10. Verfarhen nach einem der Ansprüche 1 bis 9, bei dem der Film (6) aus Schmelzklebstoff durch den Sclumelzklebstoff mit der Absorptionsschicht (8) verklebt ist

11. Verfahren nach einem der Ansprüche 1 bis 10, bei dem die Absorptionsschicht (8) ein Fluffkissen oder ein Airlaid mit oder ohne enthaltenen Superabsorber umfaßt.

12. Verfahren nach einem der Ansprüche 1 bis 11, bei dem die Absorptionsschicht Zellulosefasern und/oder Baumwollfasern und/oder Viskosefasern umfaßt.

13. Verfahren nach einem der Ansprüche 1 bis 12, bei dem auf der Außenseite der Absorptionsschicht (8) eine flüssigkeitsdurchlässige Abdeckung (9) angeordnet ist.

14. Verfahren nach Anspruch 13, bei dem die Abdeckung (9) eine Lochfolie und/oder ein perforiertes oder ein nicht perforiertes Nonwoven ist.

15. Verfahren nach einem der Ansprüche 1 bis 14, bei dem verschiedene Schichten durch Klebstoff miteinander verbunden und/oder miteinander versiegelt und/oder miteinander verprägt sind.

16. Verfahren gemäß einem der Ansprüche 1 bis 15, das eine Binde, Dickbinde, Inkontinenzeinlage, Inkontinenzvorlage, Inkontinenzpant, Slipeinlage, (Wöchnerinnen-)Vorlage, (Höschen-)Windel oder Stilleinlage ist.

## Claims

1. A method for producing a hygiene product for absorbing excretions of the human body, with a underwear protection layer (7) comprising a laminate from a nonwoven (5) and a closed, liquid-impermeable film (6) of a hot-melt-type adhesive, disposed on the inner side of the nonwoven, and an adsorption layer (8) disposed on the film (6), **characterised in that** a film (6) comprised of several layers of hot-melt-type adhesive is applied onto the inner side of the nonwoven (5), wherein the lowermost layer that has direct contact with the nonwoven penetrates into the nonwoven (5) only to a small extent due to its small weight per unit area, so that a good anchoring with the nonwoven (5) is just achieved, and the following layers melt together with the lowermost layer.

2. The method according to claim 1, wherein the film (6) of hot-melt-type adhesive has a weight per unit area of 15 g/m² at maximum.

3. A method according to claim 1 or 2, wherein the weight per unit area of the film (6) is at least 1 g/m².

4. A method according to any one of claims 1 to 3, wherein the weight per unit area of the film (6) is about 3 up to 15 g/m².

5. A method according to any one of claims 1 to 4, wherein the film (6) covers the nonwoven (5) partly or over the entire area.

6. A method according to any one of claims 1 to 5, wherein no hot-melt-type adhesive penetrated through the nonwoven (5) exists on the outer side of the nonwoven (5).

7. A method according to any one of claims 1 to 6, wherein the nonwoven (5) is a heat-bonded nonwoven (5).

8. A method according to any one of claims 1 to 7, wherein the nonwoven (5) is a spunbonded fibrous web.

9. A method according to any one of claims 1 to 8, wherein the hot-melt-type adhesive of the film (6) is a pressure-sensitive hot-melt-type adhesive.

10. A method according to any one of claims 1 to 9, wherein the film (6) of hot-melt-type adhesive is glued together with the absorption layer (8) by the hot-melt-type adhesive.

11. A method according to any one of claims 1 to 1210, wherein the absorption layer (8) comprises a fluff cushion or an airlaid with or without superabsorbent contained therein.

12. A method according to any one of claims 1 to 11, wherein the absorption layer comprises cellulose fibres and/or cotton fibres and/or viscose fibres.

13. A method according to any one of claims 1 to 12, wherein a liquid-impermeable covering (9) is disposed on the outer side of the absorption layer (8).

14. The method according to claim 13, wherein the covering (9) is a punched sheet and/or a perforated or a non-perforated nonwoven.

15. A method according to any one of claims 1 to 14, wherein different layers are bonded together and/or sealed together and/or stamped together by an adhesive.

16. A method according to any one of claims 1 to 15, which comprises a tie, thick tie, incontinence insertion pad, incontinence bed protection pad, incontinence pant, slip insert, pad for women in childbed, (panty)-diaper or breastfeeding insert.

## Revendications

1. Procédé de production d'un produit hygiénique pour absorber des excrétions du corps humain, avec une couche de protection de dessous (7) comprenant un aggloméré laminé d'un non tissé (5) et un film (6) fermé, imperméable pour liquide, d'une colle fusible disposée sur le côté intérieur du non tissé, et une couche d'absorption (8) disposée sur le film (6), **caractérisé en ce que** un film (6) de plusieurs couches de colle fusible est appliqué sur le côté intérieur du non tissé (5), la couche la plus basse qui a un contact direct avec le non tissé pénétrant seulement a minima dans le non tissé (5) à cause de son bas grammage, de sorte que un bon ancrage avec le non tissé (5) a justement lieu, et les couches suivantes se fondent avec la couche plus basse.

2. Procédé selon la revendication claim 1, dans lequel le film (6) de colle fusible a un grammage de 15 g/m² au maximum.

3. Procédé selon la revendication claim 1 or 2, dans lequel le grammage du film (6) est au moins 1g/m².

4. Procédé selon l'une quelconque des revendications 1 à 3, dans lequel le grammage du film (6) est environ 3 à 15 g/m².

5. Procédé selon l'une quelconque des revendications 1 à 4, dans lequel le film (6) recouvre le non tissé (5) partiellement ou sur la surface entière.

6. Procédé selon l'une quelconque des revendications 1 à 5, dans lequel ne pas aucune colle fusible pénétrée au travers du non tissé (5) existe sur le côté extérieur du non tissé (5).

7. Procédé selon l'une quelconque des revendications 1 à 6, dans lequel le non tissé (5) est un non tissé (5) enduit par chaleur .

8. Procédé selon l'une quelconque des revendications 1 à 7, dans lequel le non tissé (5) est un non tissé (8) filé-lié.

9. Procédé selon l'une quelconque des revendications 1 à 8, dans lequel la colle fusible du film (6) est une colle fusible sensible à la pression.

10. Procédé selon l'une quelconque des revendications 1 à 9, dans lequel le film (6) de colle fusible est collé à la couche d'absorption (8) par la colle fusible.

11. Procédé selon l'une quelconque des revendications 1 à 1210, dans lequel la couche d'absorption (8) comprend un coussin fluff ou une couche appliquée par jet d'air avec ou sans super absorbant contenu dans cela.

12. Procédé selon l'une quelconque des revendications 1 à 11, dans lequel la couche d'absorption comprend des fibres de cellulose et/ou fibres de coton et/ou fibres de viscose.

13. Procédé selon l'une quelconque des revendications 1 à 12, dans lequel une couverture imperméable pour liquides (9) est disposée sur le côté extérieur de la couche d'absorption (8).

14. Procédé selon la revendication claim 13, dans lequel la couverture (9) est une feuille à trous et/ou un non tissé perforé ou non perforé.

15. Procédé selon l'une quelconque des revendications 1 à 14, dans lequel des couches différentes sont reliées et/ou scellées et/ou gaufrées l'une à l'autre par colle.

16. Procédé selon l'une quelconque des revendications 1 à 15, qui comprend un bandeau, un bandeau épais, insert d'incontinence, drap protecteur d'incontinence, collant d'incontinence, insert de cache-sexe, drap protecteur pour accouchées, (collant)-lange ou insert de tétée.
